# EUROPEAN PATENT APPLICATION

(11) **EP 2 799 046 A1**
(43) Date of publication of application: **05.11.2014**
(21) Application number: 14165889.8
(22) Date of filing: 24.04.2014
(51) Int. Cl.: A61F 9/06

(54) **Device and method for dazzle protection**

(30) Priority: 30.04.2013 CH 8942013
(71) Applicant: OPTREL AG, 9630 Wattwil (CH)
(72) Inventor: Keller, Leo, 8630 Rüti ZH (CH); Schreiber, Martin, 8739 Rieden SG (CH); Tetzel, Tobias, 30161 Hannover (DE)
(74) Representative: Frei Patent Attorneys

(57) **Abstract**

A method is disclosed for operating an auto-darkening welding filter (1) for protecting a welder's eye in the course of a pulse mode welding process. In the course of the pulse mode welding process at least a first time interval (21, 21', 23) with strong light emission and a second time interval (22) with reduced and/or negligible light emission are repeated with a repetition rate, and a point in time of a beginning of the first time interval (21) is associated with a raising edge event (71). The method comprises the step of darkening the auto-darkening welding filter (1) to a first darkening level (51) at a point in time of the raising edge event (71) that is associated with a next first time interval (21', 23).

## Description

The invention relates to the field of dazzle or glare protection. It relates to an auto-darkening welding filter and a method for operating an auto-darkening welding filter for protecting a welder's eye in the course of a pulse mode welding process described in the preamble of the independent claims.

Advanced pulsed welding methods (e.g. MIG/MAG or TIG "Tungsten Inert Gas") use a pulse train of high current pulses, incorporating very fast rise times to quickly warm up the material. The current then is reduced to a minimum (hold current) and is followed by a lower current pulse train, which melts filler material into the liquid weld on a drop-to-drop basis. This procedure provides a very stable welding process, an improved welding efficiency under difficult welding conditions and it will allow joining different materials.

The overall repetition rate of the pulsed high current phase combined with the lower hold current phase is normally in a range of about one to 20 Hertz. State-of-the-art anti-glare protection helmets or auto-darkening welding filters (ADF) show an unsatisfactory anti-glare performance, since a pulsing welding arc is perceived, although the ADF operates in the respective protective mode. The combined delay of the opto-electronic system of these products produces an extremely disturbing, low frequency pulsing and glaring perception of the weld. Recent attempts to solve the problem (e.g. fixed scale number, or automatic adjustment of the shade between two pre-set scale numbers; e.g. US patent 8,331,001) are either not efficient enough: they use a fixed scale number or amplify the effect due to trigger delays, they have a long decay time of the LCD shutter and a slow reaction time of the transmission control circuit (two-scale number automatic adjustment). That means that the welder is extremely disturbed by the low frequency flashing nature of the pulse mode welding process.

The protective equipment has first of all, considering the high currents of up to 500 A which can be used in today's pulsed welding methods, to reach a certain darkening level quickly in order to achieve immediate protection, and to follow the low frequency pulse rate (0-20 Hz) in order to provide adequate protection and to avoid disturbance by flashing. The flashing nature of modem pulsing arc sources is responsible for the continuous overload of the visual system of the welder which is the reason that welding personnel is increasingly suffering under the disturbing and tiring effects of pulsed arc welding sources.

Current anti-glaring protection helmets are not fast enough to follow the steep, high amperage pulse train due to the combined electronic delays of the transmission control circuit. Therefore, a short flash reaches the eye and the neurological system, triggering the discomfort. In addition, commonly used LCD shutter technology employs field effect driven, but self-relaxing LC modes. These modes incorporate the important gray scale capability but produce asymmetrical switching times due to the self-relaxing decay time. The turn-on time of a normally transparent display is much faster compared to its turn-off time. The self-relaxing turn-off time does, on the other hand, not allow to follow the fast decay time of a pulsed welding arc source. This means that the liquid crystal shutter is not able to adapt from a high scale number needed in the course of high amperage pulses to a much lower scale number of the melt-off current in time.

In addition, current proposed pre-trigger concepts for anti-dazzle filters, e.g. according to patent US 6,734,393, require an interface that is specific to the respective welding equipment. The welding community does not accept such concepts as the welder does not want to have wire connections to the equipment and / or he wants to have the freedom to choose a specific welding protection helmet that is independent from the equipment. Often, different welding machines are to be used during a welding operation depending on the task to be achieved.

It is therefore an object of the invention to create an auto-darkening welding filter and a method for operating an auto-darkening welding filter for protecting a welder's eye in the course of a pulse mode welding process, which overcomes the disadvantages mentioned above and provides an improved solution to avoid or minimize disturbance of the welder by low frequency pulsing light flashes.

These objects are achieved by an auto-darkening welding filter and a method for operating such an auto-darkening welding filter according to the independent claims 1 and 9.

The method for operating an auto-darkening welding filter (ADF) is directed to protecting a welder's eye in the course of a pulse mode welding process wherein in the course of the pulse mode welding process at least a first time interval with strong light emission and a second time interval with reduced and/or negligible light emission are repeated with a repetition rate. Furthermore a point in time of a beginning of the first time interval is associated with a raising edge event, and the method comprises a step of darkening the auto-darkening welding filter to a (pre-)determined first darkening level at a point in time of the next raising edge event associated with a next first time interval, wherein the next raising edge event occurs before the beginning of the next first time interval.

The first time interval with strong light emission occurs due to a high amperage pulse train or a continuous application of a welding current in the course of the pulse mode welding process. The second time interval with reduced and/or negligible light emission occurs due to a melt-off current and/or a holding current in the course of the pulse mode welding process. The raising edge event is a point in time when an event takes place wherein in the present case the event is a sending and/or a receiving of a trigger signal for the auto-darkening welding filter. The raising and optionally also a falling edge event are automatically generated by a control system of the welding filter and cause the filter to change the darkening level of the welding filter. The raising edge event and the first time interval are correlated in time since the darkening to a first darkening level is advantageously achieved before the beginning of the first time interval eliminating dazzling of the welder. Due to the correlation between the first time interval and the raising edge event they are associated with each other. Furthemmore, the first darkening level is (pre-)determined such, that a scale number of the (pre-)determined first darkening level is high enough in order to avoid a dazzling of the welder. Therein the predetermined darkening level is a default or preset value that can be stored and taken from a memory means. The darkening level may also be adjustable to light intensity occurring in the course of the pulse mode welding process. The first darkening level is correlated with a first state of the auto-darkening welding filter.

Due to the darkening of the anti-dazzle welding filter to the first darkening level beginning at the point in time of the raising edge event of the next first time interval, the welder's eye is protected in the course of a pulse mode welding process and in particular during the first time interval of strong light emission.

The auto-darkening welding filter for protecting a welder's eye in the course of a pulse mode welding process comprises an electro-optical filter able to switch from a second state to a (pre-)determined first state upon a received raising edge event, and a control system. Thereby, the (pre-)determined first state of the electro-optical filter is associated with the first darkening level and the second state of the auto-darkening welding filter is associated with the second darkening level. The control system is configured to detect a beginning of at least a first time interval with strong light emission in the course of the pulse mode welding process, and to send a raising edge event to the electro-optical filter before a beginning of a next first time interval occurs. Thereby, the raising edge event is generated by the control system and is not received from the welding device like a conventional pre-trigger (e.g. as disclosed in US 6,734,393).

In other words one can say that due to the raising edge event a darkening signal is sent to and received by the auto-darkening welding filter. Based on such a darkening signal the electro-optical filter starts to darken at least to a first state corresponding to a first darkening level of a defined scale number. Thereby, the welder's eye is protected from strong emission of light.

The first state is characterized by a scale number which is higher than the scale number of the second state, wherein the scale number of the second state is between three and nine or up to twelve according to relevant standards. The scale number is correlated with an absorbance of the anti-dazzle welding filter, wherein a higher scale number corresponds to a higher absorbance and therefore to a lower transmittance of light through the anti-dazzle welding filter. A certain darkening level correspondently is linked to a certain defined scale number. The specified scale number is a default or preset value which can be stored and taken from a memory means. The respective attenuation corresponding to the scale number mentioned and the required welding current are correlated with each other. This correlation can be found in the relevant standards such as EN 379, respective EN 169.

The point in time of the raising edge event associated with the next first time interval can be for example estimated from the respective points in time of at least two earlier raising edge events and/or first time intervals. The at least two earlier raising edge events and/or first time intervals are used in order to determine e.g. the period or the repetition rate at which the first time intervals or the high amperage pulse trains are repeated. It is also possible to estimate the point in time of the next raising edge event from only one raising edge event in case the repetition rate is known for a particular welding device or can be estimated for example by an algorithm including experience gained during the operation of the pulse mode welding process. It is advantageous if at least two raising edge events are consecutive raising edge events.

The estimation can be carried out from determined points in time of the raising edge events and/or the first time intervals. The at least two earlier raising edge events and/or first time intervals can be consecutive raising edge events and/or first time intervals, but do not necessarily have to be consecutive.

The association of the next raising edge event with the next first time interval is related to their proximity in time, wherein the next raising edge event occurs at the same time or preferably slightly before the next first time interval. It is advantageous if the next raising edge event is shifted with respect to the beginning of the next first time interval in order to make it possible that the electro-optical filter is already darkened when the strong light emission of the first time interval or respectively of the next first time interval occurs.

A determination or estimation of the point in time of the raising edge event, typically of a sequence of such events occurring with a constant or quasi-constant repetition rate, can be based on a communication with a welding device. This communication can be established by a connection of the auto-darkening welding filter to the welding device through an interface e.g. an electrical, optical, wire-bound or wireless interface. For instance, a two-state or digital signal indicating the point in time of the rising edge of the first pulse of the high amperage pulse train can be transmitted to the auto-darkening welding filter. Thereby, the digital signal does not correspond to the raising edge event but to the beginning of the first pulse of the high amperage pulse train and therefore cannot be considered as a conventional pre-trigger.

It is also possible to determine or estimate the point in time of the raising edge event by an optical detection of the beginning of the strong light emission. For this purpose, the auto-darkening welding filter may comprise an optical detector. The optical detector is able to provide information about the point in time of the beginning of the strong light emission. Due to the above mentioned correlation of the beginning of the strong light emission and the raising edge event, information about the point in time of the raising edge event can be provided as well.

The determination or estimation of the point in time of the raising edge event and/or the first time interval can be repeated throughout the pulse mode welding process. Therefore, the repetition rate can be monitored and in case of changes a rapid or even instantaneous adaptation of the point in time of the darkening becomes possible. Thereby the welder's eyes remain continuously protected. Not only the point in time of the raising edge event or a falling edge event can be adjusted, but also the first darkening level or second darkening level can be adapted to the light intensity (brightness) emitted during the first or second time interval, respectively.

It is advantageous if the electro-optical filter or respectively a liquid crystal shutter of the auto-darkening welding filter reaches the (pre-)determined first state corresponding to the first darkening level at latest at the point in time of the beginning of the first time interval. Due to a known time delay of electro-optical filters for reaching the first darkening level, the darkening is started before the beginning of the next first time interval. This enables the auto-darkening welding filter to protect the welder's eye from glaring flashing light that originates from the high amperage pulse train of the pulse mode welding process. The starting of the darkening is initiated by the raising edge event. Therefore, it is obvious that the raising edge event is made to occur before the beginning of the next first time interval .

The point of time of darkening the auto-darkening welding filter and/or of the point of time of sending the raising edge event is shifted to precede the beginning of the associated first time interval by a predetermined time interval. Therein the predetermined time interval is a default or preset value that can be stored and taken from a memory means. The predetermined time interval ranges from one microsecond to several hundred milliseconds depending on the used liquid crystal technology.

In some cases it is advantageous if the predetermined time interval is adjustable e.g. by the welder or the control system. This enables the welder to influence the point in time when the first darkening level is reached. Thereby, a direct control of the protection can be accomplished by the welder.

Also the control system can evaluate if the welder is dazzled or if the predetermined time interval is sufficiently long in order to balance the delay of the darkening up to at least the first darkening level. It is advantageous if the predetermined time interval ensures that the first state is reached before the next first time interval occurs. This can be achieved by providing the control system with a feedback. The feedback comprises information about a light intensity behind the electro-optic shutter. The light intensity is for example measured with an optical sensor arranged at the auto-darkening welding filter, wherein the electro-optic shutter is located in between the optical sensor and the welding torch. In other words, the optical sensor is arranged behind the electro-optic shutter, and light from the welding torch reaching the optical sensor first passes through the shutter. Thereby, it is possible to operate the auto-darkening welding filter without spike signals coming from the high amperage pulse train or melt-off current of the welding device.

For example, if the optical sensor observes a brief excess of light at the beginning of the first time interval, this indicates that the shutter has closed too late. This feedback information can be used to increase, for future first time intervals, the time interval by which the raising edge event is shifted to precede the beginning of the first time interval (when the high amperage pulse train occurs).

Likewise if the optical sensor observes a brief excess of light at the end of the first time interval, this indicates that the shutter has opened up too early. This feedback information can be used to move, for future periods, the falling edge events to later points in time.

Furthermore, the first state of the electro-optical filter can be adjustable to a brightness of the first time interval. This means, that if a welding device causes brighter light or flashes in the course of the pulse mode welding process the scale number to be reached in the first state can be increased. Of course it is also possible to decrease the scale number to be reached in the first state if the light or light flash in the course of the pulse mode welding process is of less intensity, meaning less bright. In other words the auto-darkening welding filter can be adjusted such that with more intense light the electro-optical filter becomes darker (higher darkening level/higher scale number), wherein with less intense light the electro-optical filter becomes less dark (lower darkening level/smaller scale number). Such an adaptation can also be implemented for the second state, which can also be adjustable to the brightness occurring especially in the course of the second time interval, that is, between a falling edge event and the following rising edge event. Therefore the first or the second state both can be darkened states, but with different darkening levels.

Additionally, a point in time of a falling edge event can be associated with the first time interval, wherein the falling edge event occurs at about the same time as the ending of the first time interval, that is, of a high amperage pulse train. Due to such a falling edge event the darkening level of the electro-optical filter can be switched from the first darkening level to the second darkening level, wherein the second darkening level can be adjustable to the light intensity of the second time interval.

As in the case for the raising edge events, the falling edge events cause a switching of the auto-darkening welding filter from one state to another state. Thereby the switching from the first state to the second state is initiated by the falling edge event. The point in time of the falling edge event, which can occur repeatedly, can be estimated in the course of the pulse mode welding process.

Furthermore, the falling edge event can be shifted to precede or follow the end of the first time interval by a predetermined further time interval and/or in accordance with a measurement of the light passing through the filter. This is done on the one hand by moving the automatically generated falling edge events forward in time (earlier) in order to ensure that the darkening level is low enough for the welder to observe the welding process during the second time interval, e.g. a drop formation due to the melt-off current pulses. On the other hand the falling edge events may not be moved too far forward, which can be prevented by observing the light passing through the filter and not to move the falling edge event any further forward if the light increases above a limit.

Further preferred embodiments are evident from the dependent patent claims. Features of the method claims may be combined with features of the device claims and vice versa.

The subject matter of the invention will be explained in more detail in the following text with reference to exemplary embodiments which are illustrated in the attached drawings, in which:
- Figure 1a: schematically shows a characteristic curve of welding current of an advanced pulsed welding method;
- Figure 1b: schematically shows a characteristic curve of a voltage applied to a auto-darkening welding filter;
- Figure 1c: shows a characteristic curve of a scale number in the course of the advanced pulsed welding method corresponding to a darkening envelope; and
- Figure 2: shows a welding mask and torch..

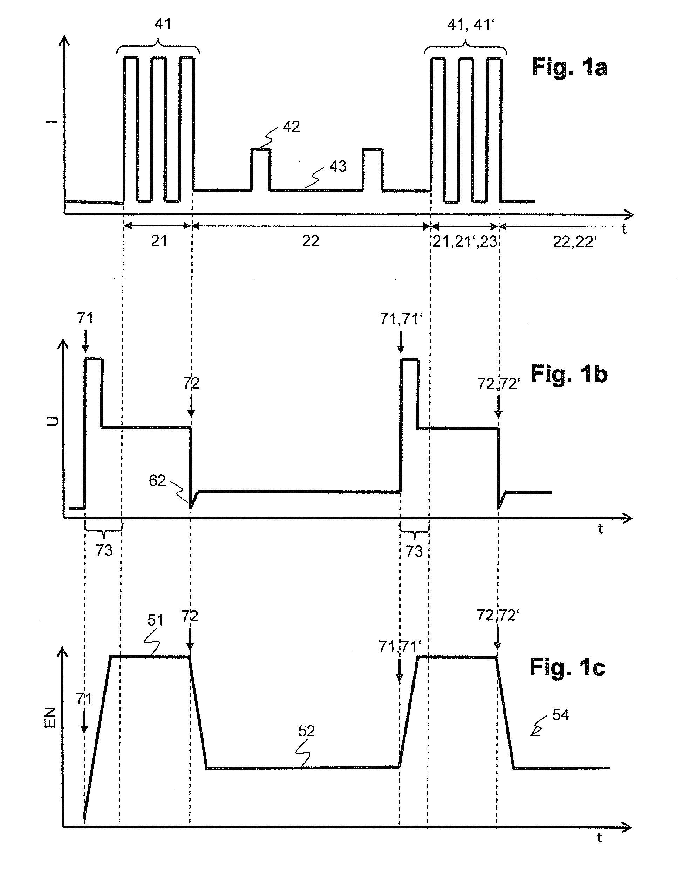

The reference symbols used in the drawings, and their meanings, are listed in summary form in the list of reference symbols. In principle, identical parts are provided with the same reference symbols in the figures. The figures 1a-c have the same time axis.

**Figure 1a** schematically shows a characteristic trajectory of a welding current of a pulse mode welding process over time. During a pulse mode welding process a high amperage pulse train 41 is provided, with e.g. the current rising up to 500 A. The high amperage pulse train 41 features a very short rise times of a rising edge and very short lowering times of a falling edge of a single pulse with a repetition rate of couple of kHz, and even more particular between 0.5 to 20 kHz, in particular between 1 to 10 kHz. The high amperage pulse train 41 can have a length of for example 180 ms, a intermediate lower current pulse train can have a length of for example 20-30 ms.

The high amperage pulse train 41 quickly heats up the material. After the high amperage pulse train 41 the current is reduced (holding current 43) and is followed by a lower current pulse train (melt-off current 42). Due to the pulses with the melt-off current 42 filler material is melted, whereas during each pulse one drop of filler material is melted. There can be, for example, one to ten such pulses in sequence, until the next high amperage pulse train occurs. Therefore, the welding can be performed on a drop-to-drop basis. The high amperage pulse trains 41 follow each other with a low frequency of e.g. 0 Hz to 30 Hz, in particular between 1-10 Hz. This procedure provides a very stable welding method, an improved welding efficiency under difficult welding conditions and it will allow joining different materials. The time period during which the high amperage pulse train 41 occurs corresponds to a first time interval 21 of the pulse mode welding process.

The length of the first time interval 21 and correspondingly the duration of the high amperage pulse train 41 can be varied within a large range by today's welding equipment, depending on welding material and parameters used. The first time interval 21 comprises a beginning of the first time interval 21 and an ending of the first time interval 21, which are illustrated by dashed lines.

**Figure 1b** shows a characteristic trajectory of a voltage applied to an electro-optical filter 11 of a anti-dazzle/auto-darkening welding filter 1 over time.

Due to the raising edge event 71 a voltage is applied to the electro-optical filter 11. The applied voltage causes a darkening of the electro-optical filter 11. In order to speed up the darkening towards a first darkening level 51 an overdriving voltage is applied right after the raising edge event 71. Furthermore, also a transition of the electro-optical filter 11 from the first darkening level 51 to the second darkening level 52 can be accelerated by a state-of-the-art short circuit technology as described in Patent Application EP 125 9852, leading to the negative peak 62. The transition of the electro-optical filter 11 to the second darkening level 52 is initiated by a falling edge event 72.

In order to avoid a disturbance of the welder the auto-darkening welding filter 1 according to the invention provides a trajectory of a scale number over time, wherein the trajectory of the scale number is also called a darkening envelope 54. The darkening envelope 54 is shown in **Figure 1c**.

In the course of the pulse mode welding process 2 the scale number always should be high enough in order that the auto-darkening welding filter 1 absorbs a sufficient fraction of the light that is emitted in the course of the pulse mode welding process 2. At the same time, the darkening envelope 54 can be adaptable to variations in different time periods/intervals (first time interval 21, second time interval 22, next first time interval 23, etc.) of the pulse mode welding process 2. Thereby, a very efficient darkening (high amplitude of darkening envelope 54) is required during the high amperage pulse train 41 in order to prevent the light of the welding arc to reach the welders eyes. In other words, the very efficient darkening is provided during a first time interval 21 with strong light emission where a first darkening level 51 has to be reached.

Furthermore, a reduced darkening (medium amplitude of the darkening envelope 54 corresponding to a second darkening level 52) is advantageous during the stage of applied holding current 43 and/or melt-off current 42 because the welder has to see the formation of the melted drop of filler material during the melt-off current 42 pulse. On the one hand the decrease of the amplitude of the darkening envelope 54 has to be fast enough to ensure that the welder can see the formation of the formed drop. In other words, the reduced darkening is provided during a second time interval 22 with reduced and/or negligible light emission where a second darkening level 52 has to be reached.

It is advantageous that the first darkening level 51 is reached at a point in time before in the course of the first time interval 21 of the pulse mode welding process 2 a strong light emission begins. In order to ensure this, the darkening of the electro-optical filter 11 starts due to a raising edge event 71 before the beginning of the first time interval 21. The raising edge event 71 is shifted to precede the beginning of the first time interval 21 by a predetermined time interval 73. The predetermined time interval 73 can be adjustable so that a welder can influence the point in time of the raising edge event 71. The predetermined time interval 73 can be a couple of microseconds, in particular 1 microsecond to 500 milliseconds. Furthermore, the raising edge event 71 is generated by the auto-darkening welding filter 1 and is not received from the welding device 12 like a conventional pre-trigger signal.

In the course of the pulse mode welding process 2 a first time interval 21 with strong light emission and a second time interval 22 with reduced and/or negligible light emission are repeated with a repetition rate. Therefore, the first time interval 21 is followed by the second time interval 22. Furthermore, the second time interval 22 is followed by a next first time interval 23. This next first time interval 23 is again followed by a next second time interval 22' and can be viewed, and processed in the same manner as the first time interval 21.

In order to avoid a disturbance of the welder the auto-darkening welding filter 1 according to the invention provides a trajectory of a scale number over time, wherein the trajectory of the scale number is also called a darkening envelope 54.

The raising edge event 71 activating the darkening of the liquid crystal shutter 11 timely corresponds to the point in time of the beginning of a rising of the darkening envelope 54. The rising of the scale number EN towards a first darkening level 51 is not instantaneous because of electronic and physical delays present in the liquid crystal shutter system 11. Due to these delays the raising edge event 71 for activating a liquid crystal shutter 11 is shifted in time slightly before the beginning of the first pulse of the high amperage pulse train 41, as shown in figure 1. Furthermore, an additional falling edge event 72 is associated with the lowering of the darkening level towards a second darkening level 52.

This lowering of the scale number EN to the second darkening level 52 is delayed in time. In order to increase the lowering speed, a short circuit of the auto-darkening welding filter 1 accelerates the self-relaxing process of the liquid crystal shutter 11. This procedure is for example known from Patent Application EP 125 9852. Nevertheless, the lowering of the darkening envelope 54 takes a certain amount of time, and therefore the additional falling edge event 72 can be transmitted to the auto-darkening welding filter 1 approximately at the same time as a the end of a last pulse of the dazzling high amperage pulse train 41. A triggering before the end of the last pulse is only needed in cases where the lowering of the darkening envelope 54 is not fast enough to ensure that the welder can see the formation of the drop of the filler material during the melt-off current 42 pulse. In case the lowering of the darkening envelope 54 is fast enough, such an early trigger event is not mandatory.

The amplitude of the darkening envelope 54 in terms of the scale number to be reached by the liquid crystal shutter 11 can be determined beforehand. Such a pre-set of the scale number is known for example from US patent 8,331,001. Alternatively, the auto-darkening welding filter 1 can automatically detect the light intensity according to the provisions laid out in patent US 8,181,270..

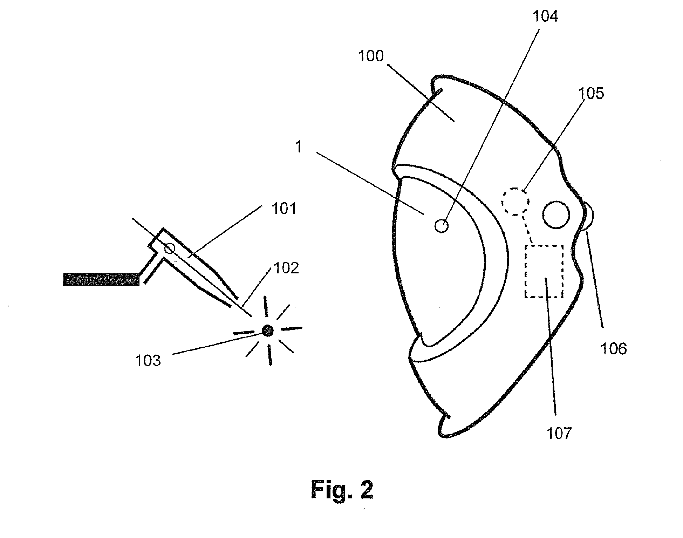

**Figure 2** schematically shows a welding mask 100 with an auto-darkening welding filter 1 (behind a transparent protective screen). The auto-darkening welding filter 1 is placed for a user to observe light 103 emitted by an electrode 102 held in a welding torch 101. The auto-darkening welding filter 1 comprises or is connected to a control system 107 with a back optical sensor 105 receiving and measuring light from the electrode 102 through the welding filter and a front optical sensor 104 receiving and measuring light from the electrode 102 (and usually ambient light as well) that has not passed through the welding filter 1. An input dial or wheel 106 can be arranged for a user to set parameters used by the control system 107, such as time delays, scale numbers etc. The arrangement of the abovementioned elements shown in the welding mask 100 is not necessarily their actual spatial arrangement. In an embodiment, the auto-darkening welding filter 1, and at least one of the control system 107, the front and back optical sensors 104, 105 and the input dial 106 or other input elements can be implemented in a single replaceable cassette.

The front optical sensor 104 can measure an intensity of light emitted from the welding process, based on which the scale number of the welding filter 1 in different states can be controlled, in particular the first darkening level 51 and the second darkening level 52. The back optical sensor 105 can measure an intensity of light passing through the welding filter 1, based on which the timing of raising edge events 71 and/or falling edge event 72 can be adjusted.

While the invention has been described in present preferred embodiments of the invention, it is distinctly understood that the invention is not limited thereto, but may be otherwise variously embodied and practiced within the scope of the claims.

## Claims

1. A **method** for operating an auto-darkening welding filter (1) for protecting a welder's eye in the course of a pulse mode welding process,
wherein in the course of the pulse mode welding process at least a first time interval (21) with strong light emission and a second time interval (22) with reduced and/or negligible light emission are repeated with a repetition rate, wherein a point in time of a beginning of the first time interval (21) is associated with a raising edge event (71),
comprising the step of:
• darkening the auto-darkening welding filter (1) to a predetermined first darkening level (51) at a point in time of a next raising edge event (71') associated with a next first time interval (21', 23), wherein the next raising edge event (71') occurs before the beginning of the next first time interval (21', 23).

2. The method according to claim 1 comprising an additional step of estimating the point in time of the next raising edge event (71') associated with the next first time interval (21', 23) from at least two earlier raising edge events (71, 71') and/or first time intervals (21, 21').

3. The method according to any of the preceding claims wherein determining of the point in time of the raising edge events (71, 71') is based on a communication with a welding device.

4. The method according to any one of claims 1 to 2 wherein determining of the point in time of the raising edge event (71, 71') is based on an optical detection of the strong light emission.

5. The method according to any one of the preceding claims wherein the point in time of the (next) raising edge event (71, 71') is determined such that the first darkening level (51) is reached at latest at the point in time of the beginning of the first time interval (21, 21', 23).

6. The method according to any one of the preceding claims wherein a point of time of darkening the anti-dazzle welding filter (1) and/or of the raising edge event (71, 71') is shifted to precede the beginning of the first time interval (21) by a predetermined time interval (73).

7. The method according to any one of claims 2-6, wherein the step of estimating the point in time of further raising edge events, each raising edge event being associated with one of further first time intervals, is repeated in the course of the pulse mode welding process.

8. The method according to claim 7, wherein the method comprises an additional step of measuring a light intensity behind the electro-optic shutter (13) and adapting the predetermined time interval (73) accordingly, in particular by, when an excess of light is measured at the beginning of the first time interval (21), advancing the raising edge events in time.

9. The method according to any one of claims 1-8, wherein a point in time of a falling edge event (72, 72') is associated with a point in time of the end of the first time interval (21, 21', 23), the method comprising the step of adjusting the auto-darkening welding filter to a second darkening level (52) due to the falling edge event (72, 72').

10. The method according to claim 9 wherein a point of time of the falling edge event (72, 72') is estimated to be near the end of the first time interval (21).

11. The method according to any one of claims 9-10, wherein the step of estimating the point in time of further falling edge events, each falling edge event being associated with one of further first time intervals, is repeated in the course of the pulse mode welding process.

12. The method according to any one of claims 9-11, wherein the method comprises an additional step of providing a feedback to the control system about a light intensity behind the electro-optic shutter (13) and adapting the timing of the falling edge events, in particular by, when an excess of light is measured at the end of the first time intervals (21), delaying the falling edge events in time.

13. An **auto-darkening welding filter** (1) for protecting a welder's eye in the course of a pulse mode welding process, comprising:
- an electro-optical filter (11) configured to switch from a second state to a first state upon receipt of a raising edge event (71, 71'), and
- a control system (107) configured to
o detect a beginning of at least a first time interval (21, 21', 23) with strong light emission in the course of the pulse mode welding process, and
o send a raising edge event (71, 71') to the electro-optical filter (11) before a beginning of a next first time interval (21', 23) occurs.

14. The auto-darkening welding filter (1) according to claim 13, wherein the point in time of the raising edge event (71, 71') sent before the beginning of the next first time interval (21', 23) is estimated from at least two earlier raising edge events and/or first time intervals (21, 21', 23).

15. The auto-darkening welding filter (1) according to any one claims 13-14, wherein the anti-dazzle welding filter (1) comprises an optical sensor (105) providing a feedback to the control system (107) about a light intensity behind the electro-optic shutter (13).

16. The auto-darkening welding filter (1) according to any one claims 13-15, configured to adjust a scale number in the second state to an emission of light in the course of the pulse mode welding process after a falling edge event (72, 72').

17. The auto-darkening welding filter (1) according to claim 16 wherein a point of time of the falling edge event (72, 72') is estimated to be near the end of the first time interval (21).

18. The auto-darkening welding filter (1) according to any one claims 13-17, configured to adjust a scale number in the first state to an emission of light in the course of the pulse mode welding process after a rising edge event (71, 71').
